(19) Europäisches Patentamt
European Patent Office
Office européen des brevets

(11) EP 0 725 786 B1

## (12) EUROPEAN PATENT SPECIFICATION

(45) Date of publication and mention
of the grant of the patent:
**12.05.1999 Bulletin 1999/19**

(21) Application number: **94926813.0**

(22) Date of filing: **13.09.1994**

(51) Int Cl.6: **C07H 11/00**, C07H 15/00,
C07H 15/20, C07H 15/22,
C07H 15/224, C07H 15/232,
C07H 15/234, C07H 17/00

(86) International application number:
**PCT/DK94/00341**

(87) International publication number:
**WO 95/07914 (23.03.1995 Gazette 1995/13)**

(54) **NOVEL SALTS OF AMINO GLYCOSIDES**

SALZE VON AMINOGLYKOSIDEN

NOUVEAUX SELS D'AMINOGLUCOSIDES

(84) Designated Contracting States:
**AT BE CH DE DK ES FR GB GR IE IT LI LU MC NL PT SE**

(30) Priority: **14.09.1993 DK 103493**

(43) Date of publication of application:
**14.08.1996 Bulletin 1996/33**

(73) Proprietor: **DUMEX-ALPHARMA A/S**
**2300 Copenhagen S (DK)**

(72) Inventors:
 • **DYRSTING, Hjarne**
**DK-2830 Virum (DK)**
 • **KOCH, Torben**
**DK-2400 Copenhagen NV (DK)**

(74) Representative: **Lindgaard, Harry et al**
**c/o Hofmann-Bang & Boutard, Lehmann &Ree A/S,**
**Hans Bekkevolds Allé 7**
**2900 Hellerup (DK)**

(56) References cited:
**EP-A- 0 403 048**

 • **ACTA PATH. MICROBIOL. IMMUNOL. SCAND. SECT. B, Volume 95, 1987, JAN JESPER ANDREASEN et al., "In Vitro Susceptibility of CampylobacterPyloridis to Cimetidine, Sucralfate, Bismuth and Sixteen Antibiotics", page 147 - page 149.**

## Description

The present invention relates to novel amino glycoside salts with sucrose-octa-O-sulfonic acid.

These novel salts may be used for the treatment of ulcerations in the stomach and/or the duodenum, preferably caused by Helicobacter pylori.

Helicobacter pylori (previously known as Campylobacter pylori) is a helical Gram-negative organism which is present in the stomach mucosa. Many recent tests have shown a clear correlation between the presence of H. pylori in the stomach mucosa and histologically shown gastritis. On the basis of this, much seems to indicate that this organism is wholly or partially responsible for the development of gastritis with ensuing ulcerations (Scand. J. Gastroenterol. 1988 (23) suppl. 142, pp. 93-100).

H. pylori is sensitive to a number of known antimicrobial substances in vitro. Furthermore, several publications disclose that the treatment of gastritis with antimicrobial agents, such as β-lactames (e.g. amoxicillin) or bismuth salts can result in the eradication of H. pylori in vivo (Antimicrobial Agents and Chemother. 1993, pp. 1184-86).

The traditional treatment of ulcerations in the human stomach or the duodenum consists in administering acid neutralising agents or preparations of the anti-histamin H2-inhibitor type which reduces the production of acid, (e.g. ranitidine, cimetidine, etc.) and acid pump-inhibitors, such as omeprazole. This treatment as such is efficient, but it has a short-term effect only as, in almost every case, there is a relapse due to the very cause of the ulcer ailment, H. pylori infection, still being present.

Today the optimum treatment of ulcerations caused by H. pylori comprise administration of bismuth subcitrate, amoxicillin and metronidazole. This treatment cures 60-90% of the patients (Ann. Rev. Med. 1992 (43) p. 142).

However, there are certain side effects associated with this therapy:

Bismuth subcitrate may cause constipation and in large doses it may be neurotoxic.

Amoxicillin and metronidazole are systemically acting antibiotics which may cause development of allergy or resistance and influence the microflora in the colon.

Therefore, there is much need for a preparation for local treatment of H. pylori infections in the stomach and the duodenum.

Amino glycosides form a group of antibiotics which have a good in vitro effect on H. pylori. The following MIC-values are known from the literature:

TABLE 1

|  | Average MIC 90 range |
|---|---|
| AMIKACIN | 0.5 |
| GENTAMYCIN | 0.04 - 1 |
| KANAMYCIN | 0.04 - 2 |
| STREPTOMYCIN | 0.04 - 1.28 |
| TOBRAMYCIN | 0.04 - 0.64 |

(Antimicrobial Agents and Chemother., 1986, pp. 510-511, J. Antimicrobial Chemother. 1986, 17, pp. 309-314, Scand, J. Gastroenterol., 1988, 23 (suppl. 142), pp. 93-100).

MIC (Minimum Inhibition Concentration) is defined as the minimum concentration expressed in mg/ml of an antimicrobial agent which is sufficient to inhibit growth of a certain percentage of the microorganisms present. Thus, MIC 90 is the concentration of antimicrobial agent sufficient to inhibit growth of 90% of the microorganisms present.

So far amino glycosides have not been found to be suitable for use in the treatment of H. pylori infections. The reason is that they are not absorbed in case of peroral administration in therapeutic doses. Amino glycosides may be administered parenterally but this is impractical in the present case as these substances have known oto- and nefro-toxic properties. When known readily soluble salts of amino glycosides are administered orally, they do not influence the stomach and duodenum mucosae due to their poor tissue penetration and thus they will not be capable of eradicating H. pylori. On the contrary, they will have a considerable influence on the colon microflora and may cause diarrhoea.

β-D-fructofuranosyl-α-D-glucopyranoside octakis (hydrogen sulfate) (in the following referred to as sucrose-octa-O-sulfonic acid) is produced by sulphating sucrose with sulphur trioxide in pyridine. In this process eight hydroxy groups in sucrose are esterified with eight molecules of sulphuric acid under formation of semiesters. Sucrose-octa-O-sulfonic acid forms well crystallized salts with Na, K, Cs, Rb and ammonium. (Chem. Pharm. Bull., 1980, 28 (2), pp. 638-341).

None of these salts have been found suitable for practical use. The only salt of the sucrose-octa-O-sulfonic acid which is used for medical therapy is the aluminium salt produced by treatment of sucrose-octa-O-sulfonic acid with aluminium hydroxide (US 3.432.489 to Chugai, 1969) and it is known under the name of sucralfate having the

gross formula $C_{12}H_{54}Al_{16}O_{75}S_8$. (Merck Index 11th Edition, pp. 1400-1401).

Sucralfate is widely used for the treatment of gastric ulcers. Peroral administration of tablets or a suspension causes sucralfate to react with the acidic gastric juice under formation of a sticky gel which adheres to the mucosa and forms a protective layer, especially on the ulcerated areas.

The effect of sucralfate is largely ascribed to the high content of aluminium hydroxide ions which act as acid neutralizers and absorb pepsin and bile salts. (Clin. Gastroenterol. 1981, 3 (suppl. 2), pp. 117-127).

No other salts of the sucrose-octa-O-sulfonic acid has hitherto been found suitable for use in the treatment of ulcer ailments.

EP 403.048 to Warner-Lambert Co. discloses mixtures of sucralfate with anti-ulcer medicaments which per se either have low solubility or, where readily soluble, are made available in a controlled-release form by means of suitable adjuncts. Among said anti-ulcer agents are antimicrobial substances, such as bismuth salts and antibiotics which may be genta-, strepto-, kana- and neomycins. Mixtures of sucralfate with these amino glycosides, however, are not mentioned in any of the examples. Only mechanically produced mixtures of sucralfate and medicaments are disclosed. No other sucrose-octa-O-sulfonic acid compositions are disclosed and a chemical interaction between the sucralfate, which as a basic aluminium salt is not capable of further salt formation, and the basic medicaments described will not be possible. Presently, there is no known method of rendering the readily soluble amino glycosides available in controlled-release form in the stomach or the duodenum. Thus, it is not possible to obtain a mechanical mixture of sucralfate and aminoglycosides having the properties necessary to fulfill the object of the invention disclosed in EP 403.048.

WO 92/18143 to Smith-Kline Beecham PLC also discloses a physical mixture of sucralfate and antibiotics of the bacteriocins-type, nisin, gramicidin and tyrothricin are particularly preferred. Nor does this publication disclose or render probable a chemical interaction between sucrose-octa-O-sulfonic acid and the basic antibiotic.

It is an object of the invention to provide novel salts of amino glycosides which are well suitable for local treatment of gastritis and/or ulcerations caused by H. pylori in the stomach and/or the duodenum of mammals including human beings.

It has surprisingly been found that sucrose-octa-O-sulfonic acid and amino glycosides form salts in stoichiometric ratios and that said salts have valuable pharmaceutical properties.

Amino glycosides belong to the group of sugars having a certain number of amino groups, preferably 4, 5 or 6. They are obtained by fermentation of various Streptomyces- or Micromonospora-species. Due to the many hydrophilic hydroxy or amino groups in the amino glycosides said substances and the salts thereof are readily soluble in water. The known salts, except from the aluminium salt, of sucrose-octa-O-sulfonic acid are also readily soluble.

As the amino glycosides have 4, 5 or 6 cation-forming amino groups and sucrose-octa-O-sulfonic acid have 8 anion-forming sulfate groups, the skilled person will not readily expect that the amino glycosides will form well defined salts with sucrose-octa-O-sulfonic acid. It should namely be taken into consideration that both amino glycosides and sucrose-octa-O-sulfonic acid have complex spatial constructions which means that the establishment of so strong ion bondings between e.g. the 4 amino groups in kanamycin A and the 8 sulfate groups in sucrose-octa-O-sulfonic acid that the resulting substance has low solubility in water is not immediately predictable.

It has now been found that most known amino glycosides having an antimicrobial activity with a content of 4 or 5 or 6 amino groups and sucrose-octa-Osulfonic acid form salts with the following general formula I:

$$([\text{sucrose-octa-O-sulfonic acid}^{8-}]\text{-}[R\text{-}(NH_3^+)_x]_y\text{-}M_z^{n+}) \qquad (I)$$

wherein

$$(x \cdot y) + (z \cdot n) = 8$$

$x \cdot y \ \in N|[4 \leq x \cdot y \leq 8 \,]$, N is the set of natural numbers,
$x \qquad \in Z|[4 \leq x \leq 6]$, Z is the set of integers,
$z \qquad \in N|[0 \leq z \leq 4]$,
$n \qquad \in Z|[1 \leq n \leq 3]$,

R is the oligosaccharide sugar moiety, preferably a mono-, di- or trisaccharide, of an amino glycoside, and M is an element capable of forming a pharmaceutically acceptable cation, preferably an element selected from the group consisting of alkaline metals, alkaline earth metals and aluminium, more preferably Na, K, Mg, Ca, Al, or $M_z^{n+}$ designates $NH_4^+$.

It has also been found that it is possible to form well defined compounds between sucrose-octa-O-sulfonic acid and an amino glycoside and then to produce salts by introducing cations. The following pharmaceutically acceptable cations are preferred:

- Alkaline metal ions, such as $Na^+$ and $K^+$, or $NH_4^+$,
- Alkaline earth metal ions, such as $Ca^{2+}$ and $Mg^{2+}$.

Another possibility is to use aluminium as an additional cation, but in this case it is more difficult to describe the resulting compounds with simple sum formulas.

The reasons are:

Normally, the Al cation is written in the following way: $Al(H_2O)_x^{3+}$, however, this ion can exist only in low concentrations. At pH values above 3, the ion is hydrolyzed to more or less soluble polynuclear forms called hydroxo complexes, polycations or hydroxo polymers.

This means, that in the cases where M in the general formula mentioned above is Al, this ion can possibly exist in more polynuclear forms as:

$$Al(H_2O)_6^{3+}, \; Al(OH)(H_2O)_5^{2+}, \; Al(OH)_2(H_2O)_4^{+}, \; Al_2(OH)_x(H_2O)_{10-x}^{6-x+},$$

$$Al_2(OH)_2(H_2O)_8^{4+}, \; or \; Al_{13}(OH)_{24}O_4(H_2O)_{12}^{7+}.$$

It is obvious that it is not possible to describe the double salts formed by sucrose-octa-O-sulfonic acid, an amino glycoside and Al in a form as exact as with cations, such as $NH_4^+$, the alkaline and alkaline earth metals listed above. Therefore the present invention is directed to all possible salts in which sucrose-octa-O-sulfonic acid, amino glycoside and Al are connected to each other by means of ion bonds.

However, in special cases it is possible to describe the new salts with Al-ions more exactly. E.g. it is well known ( J. Chem. Soc. Faraday Trans. 1, 1981, 77, p. 629-639 ) that the structural formula of sucralfate can be described as follows:

$$C_{12}H_{14}O_{11}[SO_3\text{-}Al_2(OH)_5 \, H_2O]_8$$

This means that when sucralfate dissociates the leaving Al ion is

$$Al_2(OH)_5^{+}$$

If some of the novel salts according to the invention are derived from the formula of sucralfate the novel salts can be described as follows:

$$C_{12}H_{14}O_{11}[(SO_3^{-})_8\text{-}R\text{-}(NH_3^{+})_s\text{-}(Al_2(OH)_5^{+})_t]$$

wherein $1 \leq s \leq 7$, $1 \leq t \leq 7$, and $s + t = 8$, R is oligosaccharide sugar moiety, preferably a mono-, di- or trisaccharide, of an antimicrobially active amino glycoside.

It is also possible to describe some of the novel salts in the following way:

$$([\text{sucrose-octa-O-sulfonic acid}^{8-}]\text{-}[R \text{-} (NH_3^{+})_x]_y \text{-} [Al_n(OH)_{3n-1}^{+}]_z$$

wherein

$$(x \cdot y) + z = 8$$

$x \cdot y \in Nl[1 \leq x \cdot y \leq 6]$, N is the set of natural numbers,
$x \in Zl[4 \leq x \leq 6]$, Z is the set of integers,

z $\in$ NI[$2 \leq z \leq 7$],

n $\in$ ZI[$1 \leq n \leq 5$],

R is the oligosaccharide sugar moiety, preferably a mono-, di- or trisaccharide, of an amino glycoside.

However, it should be pointed out, that it is not possible to describe all the novel double salts consisting of sucrose-octa-O-sulfonic acid, an antimicrobially active amino glycoside and Al according to the novel invention with the formulas mentioned above.

The novel salts produced according to the invention have comparatively low solubility in water. When stirred into water the novel salts form oily or sticky gels with a high degree of affinity to e.g. the inside surface of the glass container wherein the precipitation is effected. Such precipitation will also occur on biological surfaces such as the mucosae of the stomach or the duodenum.

Furthermore, the novel salts will decompose and release the amino glycoside moiety in aqueous solutions at a low pH. Therefore, the novel salts will be suitable for the treatment of ulcerations caused by H. pylori in the stomach and the duodenum.

By peroral administration in a suitable pharmaceutical formulation the novel salts will precipitate in the form of a gel which covers the mucosae of the stomach and/or the duodenum when the stomach pH is neutral or slightly below neutral.

Upon consumption of food, the stomach will secernate hydrochloric acid. H. pylori is sensitive to acid, such as gastric acid, but it has developed a protective measure. This protective measure consists in H. pylori producing an enzyme, urease, which will split urea into ammonia and CO2. The ammonia thus formed neutralizes the gastric acid.

When the areas of the stomach which are infected with H. pylori come into contact with the salts produced according to the invention, the ammonia formed will cause ion exchange with the amino glycoside portion of said salts and thus release the amino glycoside which subsequently kills the H. pylori.

If the ammonia thus formed is not sufficient to neutralize the gastric acid, a corresponding ion exchange effect will occur as the gastric acid will release the amino glycoside from the novel salts.

The novel salts of amino glycosides and sucrose-octa-O-sulfonic acid are produced in a manner known per se. They are particularly advantageously produced by allowing the aqueous solution of the amino glycoside bases to react with an aqueous solution of sucrose-octa-O-sulfonic acid by titration to obtain the desired stoichiometric ratio.

If it is desired to produce salts in a 1:1 ratio it is necessary to neutralise the excess sulfate groups in the sucrose-octa-O-sulfonic acid with suitable cations, such as $Na^+$, $Ca^+$, $Mg^{2+}$ or $Al^{3+}$. In many instances, the salts thus formed will crystallise spontaneously. If this is not the case the salt may be obtained by evaporation, optionally to dryness, or by freeze drying, or by addition of a solvent which is miscible with water, such as MeOH or EtOH.

The novel salts may also be produced by direct reaction of an aqueous solution of a salt of sucrose-octa-O-sulfonic acid with an acid addition salt of an amino glycoside.

In the special cases, where the neutralizing metal ion is Al, it is possible to operate with Al reagents, the constitution of which as mentioned before is dependent upon the chosen pH-value for the precipitation of the novel salts according to the invention. For instance, if the pH is in the range 3.5 - 4, $AlCl_3$, 6aq is dissolved in water, pH is ajusted to 3.5 - 4 with a base like NaOH or a basic ion exchange resin. In this way, $Al(OH_2)Cl$ is formed and used as mentioned in example 11 of the present application.

It is also possible to use sucralfate as the starting material. In this case, the chosen amino glycoside is dissolved in water and pH is ajusted to 4.0. To this solution, an equivalent amount of sucralfate is added with vigorous stirring. A ion exchange reaction takes place whereby the amino glycoside forms ion bonds with one or more of the sulfonic acid groups in sucrose-octa-O-sulfonic acid. and a corresponding amount of $Al(OH)_5^+$ dissolves in the aqueous phase. When the reaction is completed the novel salts are filtered off and washed with water.

This reaction will solely take place in a very narrow pH range, i.e. around pH 4. If pH is higher than 4, the Al-ion is not dissolved and, therefore, it cannot be separated from the reaction product by filtration. If pH is lower than 4, a gel formation takes place which also makes a separation impossible. Furthermore, at too low a pH, the equilibrium is moved in such a manner that the amino glycoside will remain dissolved with the Cl- as the anion.

The amino glycosides used are preferably selected from among the following substances having a desired antimicrobial activity: Amikacin, apramycin, arbekacin, bambermycins, butirosin, dibekacin, dihydrostreptomycin, fortimicin, gentamicin, isepamicin, kanamycin, micronimicin, neomycin, netilmicin, paromycin, ribostamycin, sisomicin, streptomycin, tobramycin.

Amikacin, gentamicin, kanamycin, neomycin, streptomycin and tobramycin are particularly preferred.

The novel salts according to the invention may be formulated in a known manner, e.g. in the form of tablets which may optionally be dispersible in water, capsules, powders, aqueous suspensions, syrups, etc.

It is preferred to administer the novel salts of the invention in the form of tablets or capsules which desintegrate in the stomach or duodenum and each containing from 50 to 250 mg, preferably about 100 mg of the active amino glycoside together with conventional adjuvants and/or carriers.

The invention further relates to methods of producing the compounds having the formula I as disclosed in claims 11, 12, 13 and 14, a pharmaceutical composition as described in claims 15 and 16, and a compound for use as a medicament and use of a compound for the production of a medicament as disclosed in claims 17-23.

Example 1

Sucrose-octa-O-sulfonic acid: 60 g (50 mmol) of sodium sucrose-octa-O-sulfonic acid (produced in accordance with J. Chem. Soc. Faraday Trans., 1981, 77, pp. 629-639) is dissolved in 200 ml of water and cation exchanged on Amberlite® IR 120 (H$^+$). The combined eluates are diluted to 1 liter corresponding to an 0.05 M solution.

The resulting solution is used in Examples 2 through 7.

Example 2

Kanamycin A salt of sucrose-octa-O-sulfonic acid:
17 g (25 mmol) of kanamycin A sulfate (Maiji Seika Kaisha Ltd., Tokyo, JP) is dissolved in 100 ml of ion exchanged water. The solution is eluated through an anionic exchanger, Amberlyst A26®(OH$^-$). The combined eluates are evaporated on a rotary evaporator to 100 ml and are titrated with an 0.05M solution af sucrose-octa-O-sulfonic acid (from Example 1) to a pH of 5.0. The product precipitates as a syrup. The above liquid is decanted. To the syrup is added 250 ml of EtOH and stirring is effected for 3 hours thereby effecting crystal precipitation.

The crystals are filtered off, washed with 2 x 25 ml of EtOH and vacuum dried over Sicapent at 45°C.

Yield: 20 g ∼ 82%.

DSC (Differential Scanning Calorimetry): The substance pyrolyses within the interval ranging from 180 to 195°C and exhibits no melting point.

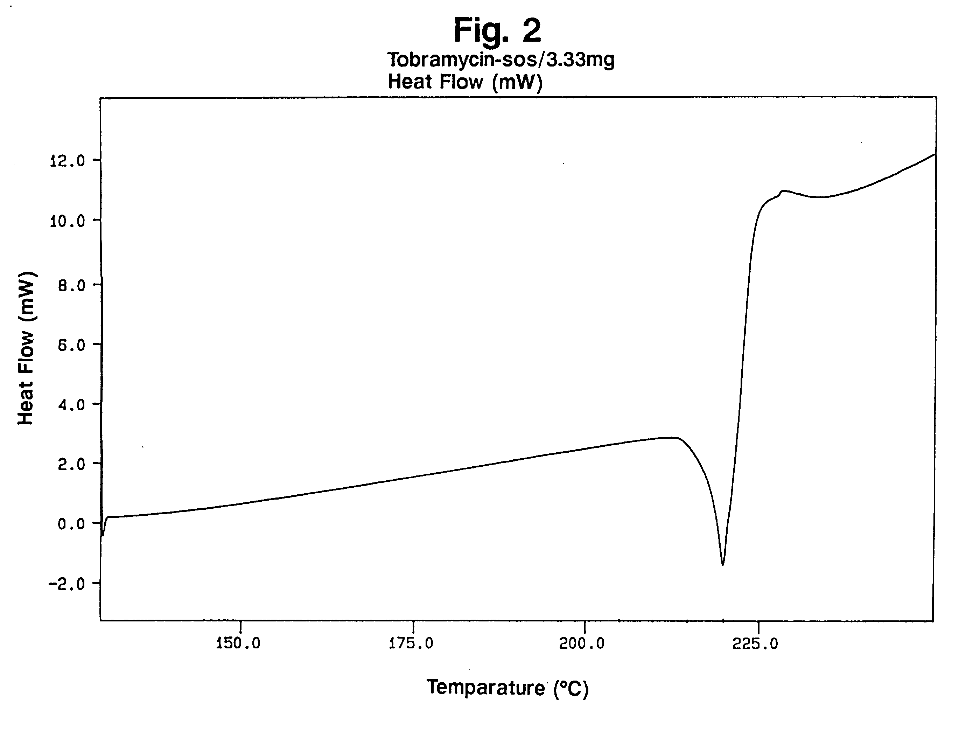

FTIR (Fourier Transformation Infra Red Spectroscopy): The spectre is almost identical to that of the substance of Example 6, cf. Figure 2.

HPLC shows that the stoichiometric composition is a kanamycin:sucrose-octa-O-sulfonic acid ratio of 2:1.

Example 2a: Small-scale dissolution test

250 mg of kanamycin salt of sucrose-octa-O-sulfonic acid in 25 ml of ion-exchanged water, 5% $NaH_2PO_4$ puffer with a pH = 3 and 0.01 M HCl, respectively, at 37±0.5°C show that the substance has low solubility in water and only dissolves by ion exchange. The solubility is thus proportional with the ion activity.

Example 3

The gentamycin salt of sucrose-octa-O-sulfonic acid:
735 mg (1 mmol) of Gentamycin sulfate (Sigma, G-3632, 648 μg/mg) is dissolved in 25 ml of ion exchanged water. The solution is eluated through an anion exchanger such as in Example 2. The combined basic eluates are titrated with an 0.05M solution af sucrose-octa-O-sulfonic acid (from Example 1) to a pH of 6.5. Evaporation to about 5 ml which are triturated with 75 ml of EtOH and stirring is maintained for three hours which results in crystal precipitation.

The crystals are separated off, washed with 2 x 10 ml of EtOH and vacuum dried over Sicapent at 45°C.

Yield 950 mg ∼ 98%.

DSC: The substance pyrolyses in the interval of 210-215°C and shows no melting point.

FTIR: Almost identical with that of the substance of Example 6, cf. Figure 2.

Example 4

The neomycin salt of sucrose-octa-O-sulfonic acid:
910 mg (1 mmol) of neomycin sulfate (Sigma, N-1876, 657 μg/mg) is dissolved in 25 ml of ion exchanged water. The solution is eluated through an anion exchanger, such as in Example 2. The combined eluates are titrated with an 0.05M solution of sucrose-octa-O-sulfonic acid (from Example 1) to a pH of 6.5. Evaporation to about 5 ml which are triturated with 75 ml of EtOH and stirring is maintained for three hours which leads to crystal precipitation.

The crystals are filtered off, washed with 2 x 10 ml of EtOH and vacuum dried over Sicapent at 45°C.

Yield 1 g ∼ 90%.

DSC: The substance pyrolyses in the interval of 190-210°C and shows no melting point.

FTIR: Almost identical with that of the substance of Example 6, cf. Figure 2.

Example 5

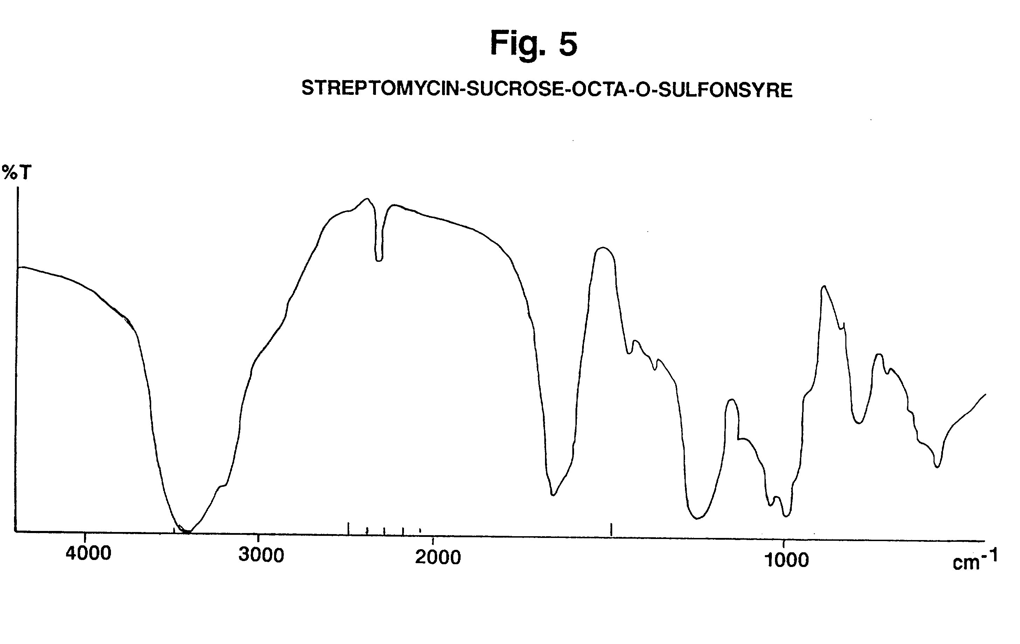

The streptomycin salt of sucrose-octa-O-sulfonic acid:
1550 mg (1 mmol) of streptomycin sulfate (Sigma, S-6501, 766 units/mg) is dissolved in 25 ml of ion exchanged water. The solution is eluated through an anion exchanger, such as in Example 2. The recovered eluates are titrated with an 0.05 M solution of sucrose-octa-O-sulfonic acid (from Example 1) to a pH of 6.5. Evaporation to about 5 ml which are triturated with 75 ml of EtOH and stirring is maintained for three hours which leads to crystal precipitation.
The crystals are filtered off, washed with 2 x 10 ml of EtOH and vacuum dried over Sicapent at 45°C.
Yield 1,5 g ~ 91%.
DSC: The substance pyrolyses in the interval of 200-210°C and shows no melting point.
FTIR: Cf. Figure 5.

Example 6

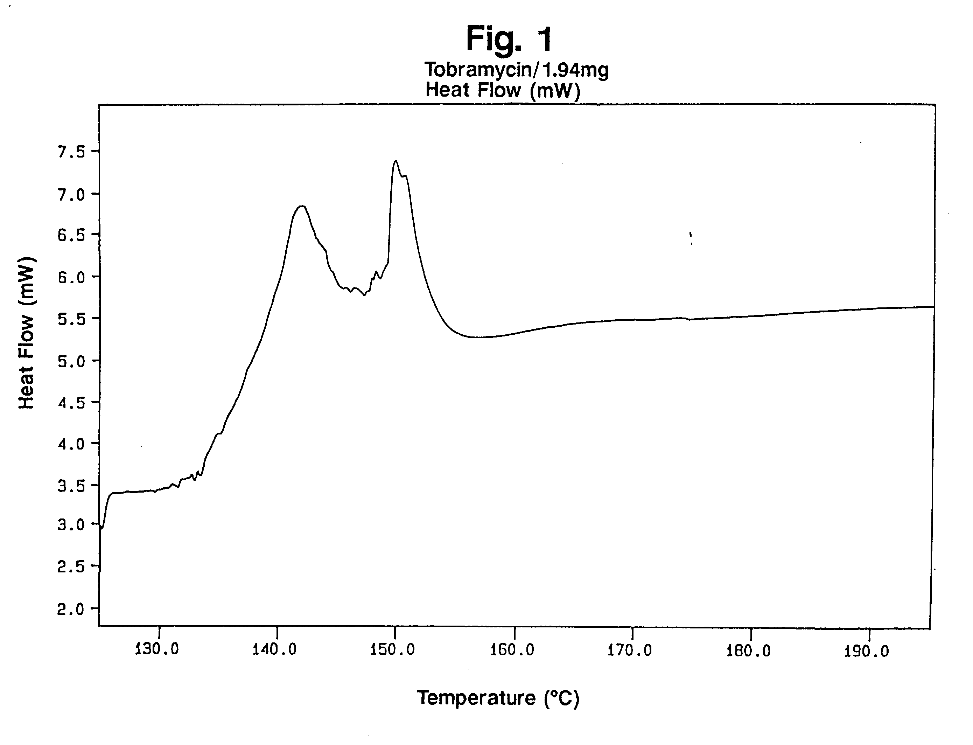

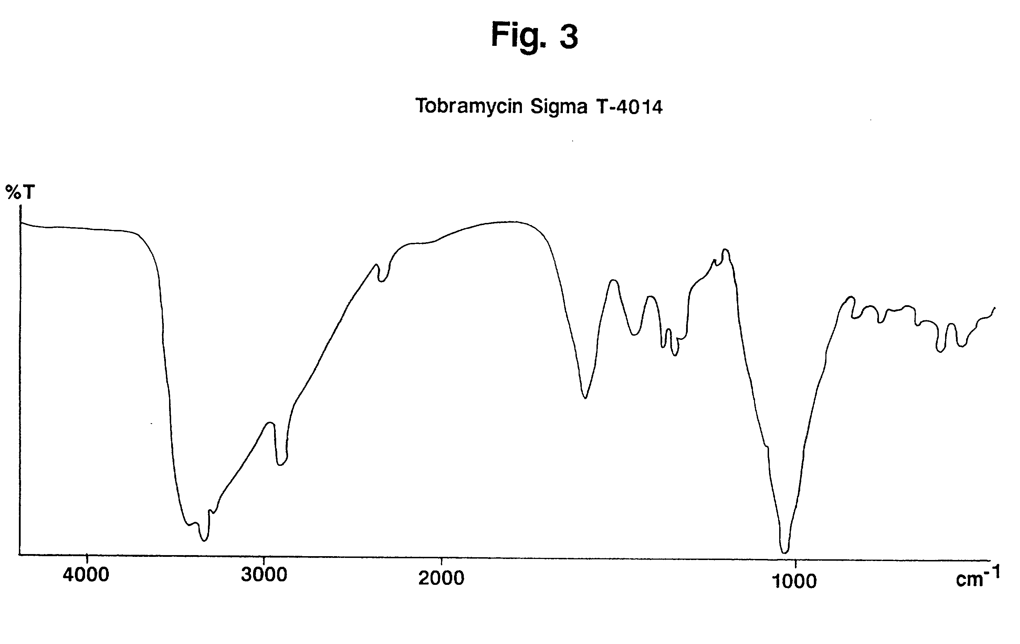

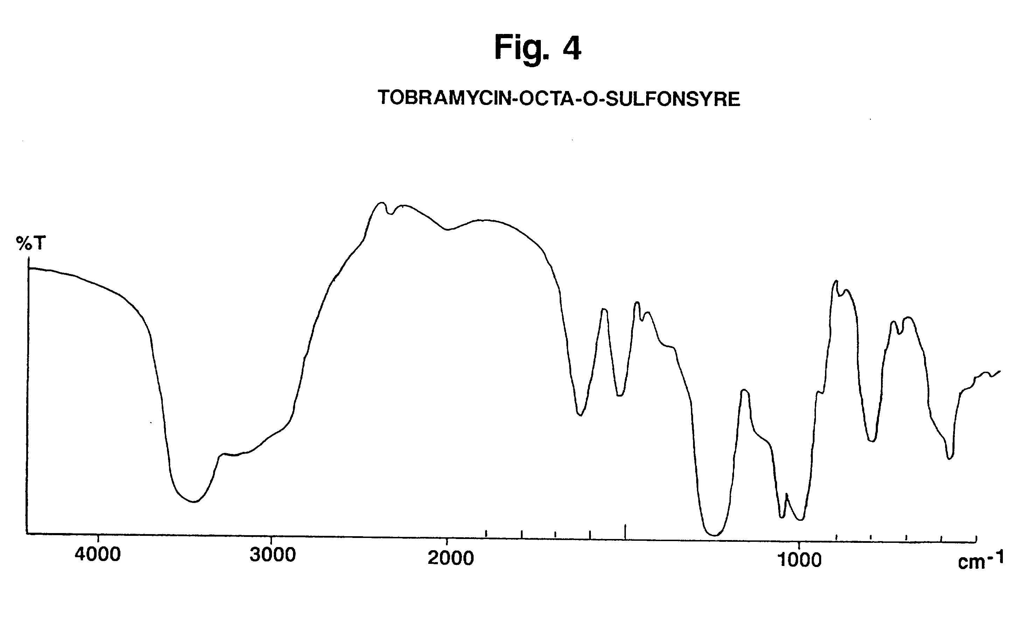

The tobramycin salt of sucrose-octa-O-sulfonic acid:
500 mg (1 mmol) of tobramycin base (Sigma, T-4014, 941 $\mu$g/mg) are dissolved in 25 ml of ion exchanged water and are titrated with an 0.05 M solution of sucrose-octa-O-sulfonic acid (from Example 1) to a pH of approximately 6.5.
The emulsion thus formed is evaporated to about 5 ml and is triturated with 75 ml of EtOH and stirring is maintained for three hours which results in crystal precipitation. The crystals are filtered off, washed with 2 x 5 ml of EtOH and vacuum dried over Sicapent at 45°C.
Yield 650 mg ~ 68%.
DSC: The substance pyrolyses in the interval of 215-230°C and shows no melting point, cf. Figure 2
DSC of tobramycin base: Cf. Figure 1.
FTIR: Cf. Figure 4.
FTIR of tobramycin base: Cf. Figure 3.
HPLC shows that the stoichiometric composition is a tobramycin:sucrose-octa-O-sulfonic acid ratio of 1.6:1.

Example 7

Kanamycin/aluminium salt of sucrose-octa-O-sulfonic acid.
Is produced by admixing equimolar amounts of sucrose-octa-O-sulfonic acid with kanamycin A-base. pH is subsequently adjusted to 5.2 with a slurry of $Al(OH)_3$ in water. The resulting gel is evaporated in a rotation evaporator to form an amorphous powder.
The production of corresponding salts with NaOH, $Ca(OH)_2$ and $Mg(OH_2)$ is carried out analogously with Example 7.
The following applies to all of the substances of Examples 2 through 7:
The produced reaction with water is a swelling reaction whereby a sticky substance or syrup is formed. The absorption of water occurs to a certain point where the particles have become an oily, viscuous fluid which has comparatively low solubility in water. When applying such a drop on smooth skin a membrane is formed which has low solubility in water and which may only be removed by intensive scrubbing with water. By rinsing with ion-containing water the membrane is gradually dissolved until it disappears completely.

Example 8

The production of kanamycin salt of sucrose-octa-O-sulfonic acid with Na-sucrose-octa-sulfate as starting substance.
7.75 g (10 mmol) of kanamycin A-sulfate dissolved in 50 ml of water is admixed with 6.5 g (5 mmol) of sodium sucrose-octa-sulfate, $C_{12}H_{14}S_8O_{35}Na_8$, $8H_2O$, produced in accordance with Example 1.
The mixture is dialysed until the ion strength of the washing water is constant, at this point kanamycin-octa-sulfate is dissolved, and the solution becomes milky.
The emulsion is evaporated to about 25 ml and triturated with 250 ml of ethanol whereby crystallisation occurs. Stirring is maintained for 3 hours and crystals are filtered off and dried as described under Example 2.
Yield 9,55 g ~ 98%.
Analysis of the substance gives the same results as under Example 2.

Example 9

Sucrose-octa-O-sulfonic acid-Al-tobramycin 5.6% salt.

1) 5 mmol sucrose-octa-O-sulfonic-acid-tobramycin$_{1.6}$ (prepared in ex. 6) and 7 mmol Na-sucrose-octa-O-sulfate is dissolved in 150 ml water.

2) 112 mmol AlCl$_3$ in 100 ml water is mixed with 224 mmol NaOH in 100 ml water.

Solution 1 and 2 are mixed with stirring. pH is ajusted to and maintained at 5.5 with 0.2 M NaOH, whereby a precipitate is formed.

The precipitate is filtered off, washed with 4 x 25 ml of water and dried in vacuum oven at 25°C/P$_4$O$_{10}$.

Yield: 75%.

The content of tobramycin is analyzed by HPLC to be 5,6%.

Example 10

Test for bioadhesion on mucosal surfaces.

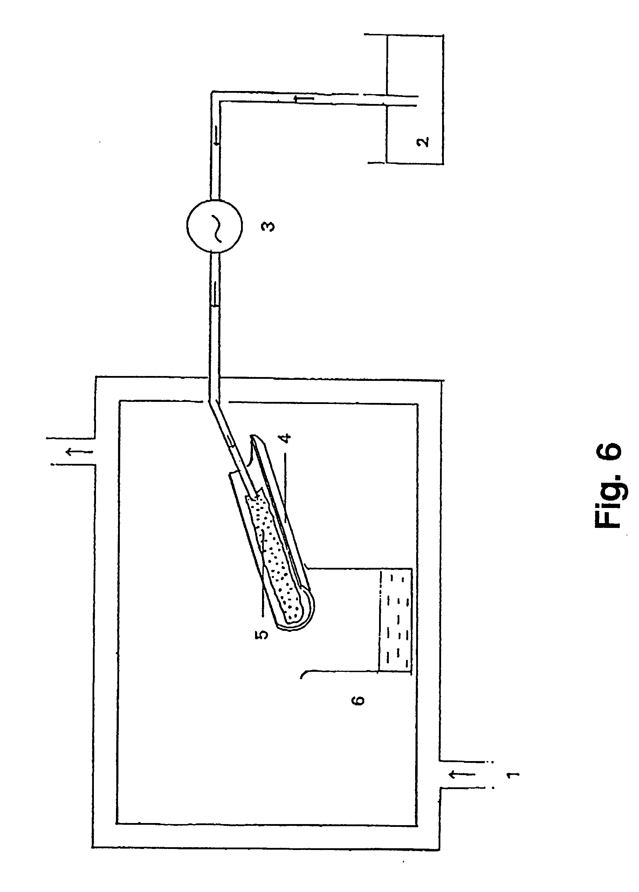

The bioadhesion of the novel salts according to the invention is demonstrated in a test system shown on Fig. 6. wherein (1) is thermostatic water flow at 40°C, (2) is a reservoir containing the washing solution at 37°C, (3) is a peristaltic pump (4) is a stainless steel support, (5) is a model membrane, and (6) is a receiver for collecting the washings.

A segment of jejunum from rabbit, is placed on a support (4) with the mucosa facing upwards, spread and held in position on the support by the adhesive effect of the jejunum itself. Support and jejunum is placed at an angle of -7° in a cylindrical cell thermostated at 37°C. The sample to be tested is placed on the jejunum, 1 ml buffer is dropped thereon. The buffers used is either a 0.01 M HCl solution, pH 2 (buffer a) or borate buffer solution 0.05 M, pH 7.4 (buffer b). Now the segments are left for 10 minutes in the cell to allow the test substance to interact with the glycoproteins of the mucosa. After 10 minutes the jejunum is flushed with either buffer a or b for 30 minutes.

The buffer is collected in a receiver (6). The amount of test substance remaining on the jejunum is calculated by measuring the amount in the receiver by means of HPLC.

In a first experiment, the tobramycin-SOS salt from example 6 containing 44% tobramycin, was tested. As a reference, a physical mixture of sucralfate 56%/tobramycin 44% was used.

The following results were obtained:

| Test substance | Buffer a, pH 2.0 | Buffer b, pH 7.4 |
|---|---|---|
| SOS-tobramycin salt from ex. 6 | 59% | 31% |
| SOS/tobramycin physical mixture 56:44 | 8% | 1% |

The percentages indicates the amount of test substance remaining on the mucosa.

In a second experiment, the following results were obtained:

| Test substance | Buffer a, pH 2.0 | Buffer b, pH 7.4 |
|---|---|---|
| SOS-Al-tobramycin salt from ex. 9 | 61% | 65% |
| Sucralfate 94.4%-tobramycin 5.6% physical mixture | 40% | 50% |

The percentages indicate the amount of test substance remaining on the mucosa.

These experiments clearly demonstrates, that the novel salts according to the invention are superior to simple physical mixtures of sucralfate and tobramycin.

Example 11.

Preparation of salts of sucrose-octa-O-sulfonic acid-tobramycin-Al.

I: Preparation of Al(OH)$_2$Cl-reagent (I):

0.2 mol AlC$_3$,6 aq, ~ 48.2 g is dissolved in 200 ml demineralized water and is ajusted to pH 3.5-4 with 0.4 mol NaOH - 200 ml 2 M NaOH. The solution is metastable and is kept at +5°C.

II: Preparation of $Al(OH)_2Cl$ reagent (II):

0.2 mol $AlCl_3$,6 aq, ~ 48.2 g is dissdlved in 300 ml demineralized water and ajusted to pH = 3.9 with a strong basic ion exchange reagent, eg. Amberlite® A-26 in hydroxy-form. The ajustment is done over 5 minutes with moderate stirring. The anion exchange reagent is filtered off and eluated with 200 ml of water for 30 minutes. The combined filtrate and eluate are filtered through Celite® (diatomaceous earth).

The solution is metastable and is kept at + 5°C.

III:

100 ml, 0.1 M ~ 10 mmol $SOSH_8$, prepared in example 1 is mixed with 8.5 g tobramycin base, dissolved in 50 ml water. The mixture is stirred for 10 minutes and 400 ml 0.5 M $Al(OH)_2$-reagent (I) is added ~ 200 mmol $A)(OH)_2^+$. Stirring is continued for 10 minutes, 100 ml 2 M NaOH ~ 200 mmol is added which results in the formation of a precipitate. Stirring is continued for 2 minutes and the crystals are filtered off on a glass filter, washed with 50 ml water, and dried in vacuum oven at 80°C/133,3 Pa (1 Torr)/20 hours.

Yield ~ 35 g, 73%.

IV;

Same procedure as III, but by use of reagent II.

Yield: 36 g ~ 75%.

**Claims**

1. A compound having the general formula I:

$$([\text{sucrose-octa-O-sulfonic acid}^{8-}]\text{-}[R\text{-}(NH_3^+)_x]_y\text{-}M_z^{n+}) \qquad (I)$$

wherein

$$(x \cdot y) + (z \cdot n) = 8$$

$x \cdot y \quad \in NI[4 \leq x \cdot y \leq 8]$, N is the set of natural numbers,

$x \quad \in ZI[4 \leq x \leq 6]$, Z is the set of integers,

$z \quad \in NI[0 \leq z \leq 4]$,

$n \quad \in ZI[1 \leq n \leq 3]$,

R is the oligosaccharide sugar moiety, preferably a mono-, di- or trisaccharide, of an amino glycoside and M is an element capable of forming a pharmaceutically acceptable cation, preferably an element selected from the group consisting of alkaline metals, alkaline earth metals and aluminium, more preferably Na, K, Mg, Ca, Al, or $M_z^{n+}$ designates $NH_4^+$.

2. A compound according to claim 1, wherein M is an element selected from the group consisting of alkaline metals, alkaline earth metals and aluminium.

3. A compound according to claim 1, wherein the amino glycoside is selected from the group consisting of kanamycin A, gentamicin, neomycin, netilmicin, streptomycin, tobramycin.

4. A compound according to any one of claims 1, 2 or 3 selected from among compounds having the following formulas:

[sucrose-octa-O-sulfonic acid] - [kanamycin A]$_2$ ,

[sucrose-octa-O-sulfonic acid] - [gentamicin]$_2$,

[sucrose-octa-O-sulfonic acid] - [neomycin]$_{1.3}$,

[sucrose-octa-O-sulfonic acid] - [netilmycin]$_{1.6}$,

[sucrose-octa-O-sulfonic acid] - [streptomycin]$_{1.6}$,

[sucrose-octa-O-sulfonic acid] - [tobramycin]$_{1.6}$,

and pharmaceutically acceptable salts thereof.

5. A compound according to claim 1 in the form of a salt of sucrose-octa-O-sulfonic acid with an antimicrobially active aminoglycoside and with aluminium.

6. A compound according to claim 5 in which the chemical bonds between sucrose-octa-O-sulfonic acid, an aminoglycoside and aluminium are ion bonds.

7. A composition according to the formula:

$$([\text{sucrose-octa-O-sulfonic acid}^{8-}]\text{-}[R\text{-}(NH_3^+)_x]_y\text{-}[Al_n(OH)_{3n-1}^+]_z),$$

wherein

$$(x \cdot y) + z = 8$$

$x \cdot y \quad \in N|[1 \leq x \cdot y \leq 6]$, N is the set of natural numbers,
$x \quad \in Z|[4 \leq x \leq 6]$, Z is the set of integers,
$z \quad \in N|[2 \leq z \leq 7]$,
$n \quad \in Z|[1 \leq n \leq 5]$,

wherein R is the oligosaccharide sugar moiety, preferably a mono-, di- or trisaccharide, of an antimicrobially active amino glycoside.

8. A composition according to claim 1 of the formula:

$$(C_{12}H_{14}O_{11}[(SO_3^-)_8\text{-}R\text{-}(NH_3^+)_s\text{-}(Al_2(OH)_5^+)_t]),$$

wherein $1 \leq s \leq 7$, $1 \leq t \leq 7$, and $s + t = 8$, R is oligosaccharide sugar moiety, preferably a mono-, di- or trisaccharide, of an antimicrobially active amino glycoside.

9. A compound according to claim 5, 6, 7, or 8 wherein the amino glycoside is selected from the group consisting of kanamycin A, gentamicin, neomycin, netilmicin, streptomycin, tobramycin.

10. A compound according to claim 5, 6, 7, or 8 wherein the amino glycoside is tobramycin.

11. A method of producing the compounds according to claims 1 through 4, wherein an aqueous solution of amino glycoside base is reacted with an aqueous solution of sucrose-octa-O-sulfonic acid.

12. A method according to claim 11, wherein cations, preferably $K^+$, $Na^+$, $Mg^{2+}$, $Ca^{2+}$ and $Al^{3+}$, are introduced to obtain salts that are pharmaceutically acceptable.

13. A method of producing the compounds according to claim 1 through 4, wherein an aqueous solution af an acid addition salt of an amino glycoside is reacted with an aqueous solution of a salt of sucrose-octa-O-sulfonic acid followed by dialysis for removal of the inorganic salts.

14. A method of producing a compound according to claims 5 through 10, wherein an aqueous suspension of sucralfate is reacted with an amino glycoside in water at a pH of about 4.

15. A pharmaceutical composition containing at least one compound having the general formula I comprising from 50 to 250 mg of an antimicrobially active amino glycoside per unit dose, and a pharmaceutically acceptable carrier.

16. A pharmaceutical composition containing at least one compound according to claim 5 comprising from 50 to 250 mg of an antimicrobially active amino glycoside per unit dose, and a pharmaceutically acceptable carrier.

17. A compound for treating gastritis and ulcerations in the stomach and/or the duodenum in a subject in need of such treatment, the said compound having the general formula I:

$$([\text{sucrose-octa-O-sulfonic acid}^{8-}] - [R\text{-}(NH_3^+)_x]_y\text{-}M_z^{n+}) \tag{I}$$

wherein

$$(x \cdot y) + (z \cdot n) = 8$$

$x \cdot y \quad \in NI[4 \leq x \cdot y \leq 8]$, N is the set of natural numbers,
$x \quad \in ZI[4 \leq x \leq 6]$, Z is the set of integers,
$z \quad \in NI[0 \leq z \leq 4]$,
$n \quad \in ZI[1 \leq n \leq 3]$,

wherein R is the oligosaccharide sugar moiety, preferably a mono-, di- or trisaccharide, of an amino glycoside and M is an element capable of forming a pharmaceutically acceptable cation, preferably an element selected from the group consisting of alkaline metals, alkaline earth metals and aluminium, more preferably Na, K, Mg, Ca, Al, or $M_z^{n+}$ designates $NH_4^+$ and pharmaceutically acceptable salts thereof, which compound is effective for such purpose.

18. A compound according to claim 5 and pharmaceutically acceptable salts thereof for treating gastritis and ulcerations in the stomach and/or the duodenum in a subject in need of such treatment, which compound is effective for such purpose.

19. A compound for treating gastritis and ulcerations in the stomach and/or the duodenum in a subject in need of such treatment, the said compound having the general formula:

$$([\text{sucrose-octa-O-sulfonic acid}^{8-}]\text{-}[R\text{-}(NH_3^+)_x]_y\text{-}[Al_n(OH)_{3n-1}^+]_z),$$

wherein

$$(x \cdot y) + z = 8$$

$x \cdot y \quad \in NI[1 \leq x \cdot y \leq 6]$, N is the set of natural numbers,
$x \quad \in ZI[4 \leq x \leq 6]$, Z is the set of integers,
$z \quad \in NI[2 \leq z \leq 7]$,
$n \quad \in ZI[1 \leq n \leq 5]$,

wherein R is the oligosaccharide sugar moiety, preferably a mono-, di- or trisaccharide, of an antimicrobially active amino glycoside, and pharmaceutically acceptable salts thereof which compound is effective for such purpose.

20. A compound for treating gastritis and ulcerations in the stomach and/or the duodenum in a subject in need of such treatment, the said compound having the formula:

$$(C_{12}H_{14}O_{11}[(SO_3^-)_8\text{-}R\text{-}(NH_3^+)_s\text{-}(Al_2(OH)_5^+)_t]),$$

wherein $1 \le s \le 7$, $1 \le t \le 7$, and $s + t = 8$, R is oligosaccharide sugar moiety, preferably a mono-, di- or trisaccharide, of an antimicrobially active amino glycoside.

21. A compound according to claims 19, 20, 21 or 22 wherein said compound is in the form of a pharmaceutical composition comprising from 50 to 250 mg of an antimicrobially active amino glycoside per unit dose together with a pharmaceutically acceptable carrier or diluent.

22. Use of a compound according to any one of claims 1 to 10 for the preparation of a medicament suitable for the treatment of gastritis and ulcerations in the stomach and the duodenum in mammals.

23. Use of a compound according to any one of claims 1 to 10 for the preparation of a medicament suitable for the eradication of Helicobacter pylori in the stomach and duodenum of a mammal.

**Patentansprüche**

1. Verbindung der allgemeinen Formel I;

$$([\text{Sucrose-octa-O-sulfonsäure}^{8-}]\text{-}[R\text{-}(NH_3^+)_x]_y\text{-}M_z^{n+}) \qquad \text{(I)}$$

wobei

$$(x \cdot y) + (z \cdot n) = 8$$

ist,

$x \cdot y \in NI[4 \le x \cdot y \le 8]$, wobei N die Reihe der natürlichen Zahlen ist,
$x \in ZI[4 \le x \le 6]$, wobei Z die Reihe der ganzen Zahlen ist
$z \in NI[0 \le z \le 4]$,
$n \in ZI[1 \le n \le 3]$,

R eine Oligosaccharidzuckereinheit, bevorzugt ein Mono-, Di- oder Trisaccharid eines Aminoglycosids, ist, und M zur Bildung eines pharmazeutisch verträglichen Kations geeignet ist, bevorzugt ausgewählt aus Alkalimetallen, Erdalkalimetallen und Aluminium, stärker bevorzugt aus Na, K, Mg, Ca und Al, ist, oder $M_z^{n+}$ $NH_4^+$ bezeichnet.

2. Verbindung nach Anspruch 1, wobei M ausgewählt aus Alkalimetallen, Erdalkalimetallen und Aluminium ist.

3. Verbindung nach Anspruch 1, wobei das Aminoglycosid ausgewählt aus Kanamycin A, Gentamicin, Neomycin, Netilmicin, Streptomycin und Tobramycin ist.

4. Verbindung nach einem der Ansprüche 1, 2 oder 3, ausgewählt aus den Verbindungen der folgenden Formeln:

$$[\text{Sucrose-octa-O-sulfonsäure}] - [\text{Kanamycin A}]_2,$$

$$[\text{Sucrose-octa-O-sulfonsäure}] - [\text{Gentamicin}]_2,$$

[Sucrose-octa-O-sulfonsäure] - [Neomycin]$_{1,3}$,

[Sucrose-octa-O-sulfonsäure] - [Netilmicin]$_{1,6}$,

[Sucrose-octa-O-sulfonsäure] - [Streptomycin]$_{1,6}$,

[Sucrose-octa-O-sulfonsäure] - [Tobramycin]$_{1,6}$,

und deren pharmazeutisch verträglichen Salzen.

**5.** Verbindung nach Anspruch 1 in der Form eines Salzes von Sucrose-octa-O-sulfonsäure mit einem antimikrobiell wirksamen Aminoglycosid und mit Aluminium.

**6.** Verbindung nach Anspruch 5, wobei die chemischen Bindungen zwischen Sucrose-octa-O-sulfonsäure, einem Aminoglykosid und Aluminium ionische Bindungen sind.

**7.** Verbindung der Formel:

$$([\text{Sucrose-octa-O-sulfonsäure}^{8-}]\text{-}[R\text{-}(NH_3^+)_x]_y\text{-}[Al_n(OH)_{3n-1}{}^+]_z,$$

in der

$$(x \cdot y) + z = 8$$

$x \cdot y \in N|1{\leq}x{\cdot}y{\leq}6]$, N die Reihe der natürlichen Zahlen ist
$x \quad \in Z|[4{\leq}x{\leq}6]$, Z die Reihe der ganzen Zahlen ist,
$z \quad \in N|[2{\leq}z{\leq}7]$,
$n \quad \in Z|[1{\leq}n{\leq}5]$,

wobei R eine Oligosaccharidzuckereinheit, bevorzugt ein Mono-, Di- oder Trisaccharid eines antimikrobiell wirksamen Aminoglycosids ist.

**8.** Verbindung nach Anspruch 1 der folgenden Formel:

$$C_{12}H_{14}O_{11}[(SO_3^-)_8\text{-}R\text{-}(NH_3^+)_s(Al_2(OH)_5{}^+)_t] ,$$

wobei $1{\leq} s {\leq}7$, $1{<}t{\leq}7$ und $s + t = 8$, R eine Oligosaccharidzuckereinheit, bevorzugt ein Mono-, Di- oder Trisaccharid eines antimikrobiell wirksamen Aminoglycosids, ist.

**9.** Verbindung nach einem der Ansprüche 5, 6, 7 oder 8, wobei das Aminoglycosid aus Kanamycin A, Gentamicin, Neomycin, Netilmicin, Streptomycin und Tobramycin ausgewählt ist.

**10.** Verbindung nach einem der Ansprüche 5, 6, 7 oder 8, wobei das Aminoglycosid Tobramycin ist.

**11.** Verfahren zur Herstellung der Verbindungen gemäß der Ansprüche 1 bis 4, wobei eine wäßrige Lösung einer Aminoglycosidbase mit einer wäßrigen Lösung von Sucrose-octa-O-sulfonsäure umgesetzt wird.

**12.** Verfahren nach Anspruch 11, wobei Kationen, bevorzugt K$^+$, Na$^+$, Mg$^{2+}$, Ca$^{2+}$ und Al$^{3+}$, eingeführt werden, um pharmazeutisch verträgliche Salze zu erhalten.

**13.** Verfahren zur Herstellung der Verbindungen gemäß der Ansprüche 1 bis 4, wobei die wäßrige Lösung eines Säureadditionssalzes eines Aminoglycosids mit einer wäßrigen Lösung eines Salzes von Sucrose-octa-O-sulfonsäure umgesetzt und anschließend die anorganischen Salze durch Dialyse entfernt wird.

14. Verfahren zur Herstellung einer Verbindung gemäß der Ansprüche 5 bis 10, wobei eine wäßrige Suspension von Sucrosesulfat mit einem Aminoglycosid in Wasser bei einem pH-Wert von ungefähr 4 umgesetzt wird.

15. Arzneimittel, enthaltend mindestens eine Verbindung der allgemeinen Formel I, umfassend 50 bis 250 mg eines antimikrobiell wirksamen Aminoglycosids pro Dosiseinheit und einen pharmazeutisch verträglichen Träger.

16. Arzneimittel enthaltend mindestens eine Verbindung gemäß Anspruch 5, umfassend 50 bis 250 mg eines antimikrobiell wirksamen Aminoglycosids pro Dosiseinheit und einen pharmazeutisch verträglichen Träger.

17. Verbindung zur Behandlung von Gastritis und Geschwüren im Magen und/oder im Duodenum bei einem Menschen oder Tier, die solch eine Behandlung benötigen, wobei die Verbindung die allgemeine Formel I aufweist:

$$[\text{Sucrose-octa-O-sulfonsäure}^{8-}]\text{-}[R\text{-}(NH_3^+)_x]_y\text{-}M_z^{n+}) \tag{I}$$

wobei

$$(x \cdot y) + (z \cdot n) = 8$$

$x \cdot y \quad \in NI[4 \leq x \cdot y \leq 8]$, N die Reihe der natürlichen Zahlen ist,
$x \quad \in ZI[4 \leq x \leq 6]$, Z die Reihe der ganzen Zahlen ist,
$z \quad \in NI[0 \leq z \leq 4]$,
$n \quad \in ZI[1 \leq n \leq 3]$,

wobei R eine Oligosaccharidzuckereinheit, bevorzugt ein Mono-, Di- oder Trisaccharid eines .Aminoglycosids ist und M zur Bildung eines pharmazeutisch verträglichen Kations geeignet ist, bevorzugt ausgewählt aus Alkalimetallen, Erdalkalimetallen und Aluminium, stärker bevorzugt aus Na, K, Mg, Ca und Al, oder $M_z^{n+}$, bezeichnend $NH_4^+$ und pharmazeutisch verträgliche Salze, ist, und wobei die Verbindung für solche Zwecke wirksam ist.

18. Verbindung nach Anspruch 5 und deren pharmazeutisch verträgliche Salze zur Behandlung von Gastritis und Geschwüren im Magen und/oder im Duodenum bei einem Menschen oder Tier, falls eine solche Behandlung erforderlich ist, wobei die Verbindung für solche Zwecke wirksam ist.

19. Verbindung zur Behandlung von Gastritis und Geschwüren im Magen und/oder im Duodenum bei einem Menschen oder Tier, falls eine solche Behandlung erforderlich ist, wobei die Verbindung folgende allgemeine Formel aufweist:

$$[\text{Sucrose-octa-O-sulfonsäure}^{8-}]\text{-}[R\text{-}(NH_3^+)_x]_y\text{-}[Al_n(OH)_{3n\text{-}1}^+]_{z'}$$

wobei

$$(x \cdot y) + z = 8$$

$x \cdot y \quad \in NI[1 \leq x \cdot y \leq 6]$, N die Reihe der natürlichen Zahlen ist,
$x \quad \in ZI[4 \leq x \leq 6]$, Z die Reihe der ganzen Zahlen ist,
$z \quad \in NI[2 \leq z \leq 7]$,
$n \quad \in ZI[1 \leq n \leq 5]$,

wobei R eine Oligosaccharidzuckereinheit, bevorzugt ein Mono-, Di- oder Trisaccharid eines antimikrobiell wirksamen Aminoglycosids und dessen pharmazeutisch verträgliche Salze, ist, wobei die Verbindung für solche Zwecke wirksam ist.

20. Verbindung zur Behandlung von Gastritis und Geschwüren im Magen und/oder im Duodenum bei einem Menschen oder Tier, falls eine solche Behandlung erforderlich ist, wobei die Verbindung folgende Formel aufweist:

$$C_{12}H_{14}O_{11}[(SO_3^-)_8\text{-}R\text{-}(NH_3^+)_s(Al_2(OH)_5^+)_t],$$

wobei $1 \leq s \leq 7$, $1 \leq t \leq 7$, und $s + t = 8$, R eine Oligosaccharidzuckereinheit, bevorzugt ein Mono-, Di- oder Trisaccharid eines antimikrobiell wirksamen Aminoglycosids, ist.

21. Verbindung nach den Ansprüchen 19, 20, 21 oder 22, wobei die Verbindung ein Arzneimittel ist, umfassend 50 bis 250 mg eines antimikrobiell wirksamen Aminoglycosids pro Dosiseinheit zusammen mit einem pharmazeutisch verträglichen Träger, oder einem Verdünnungsmittel.

22. Verwendung einer Verbindung gemäß einem der Ansprüche 1 bis 10 zur Herstellung eines zur Behandlung von Gastritis und Geschwüren im Magen und im Duodenum von Säugern geeigneten Medikaments.

23. Verwendung einer Verbindung gemäß einem der Ansprüche 1 bis 10 zur Herstellung eines zur Ausrottung von Helicobacter pylori im Magen und Duodenum von Säugern geeigneten Medikaments.

## Revendications

1. Composé présentant la formule générale I suivante :

$$([\text{acide sucrose-octa-O-sulfonique}^{8-}] \text{ - } [R\text{-}(NH_3^+)_x]_y\text{-}M_z^{n+}) \tag{I}$$

dans laquelle

$$(x \bullet y) + (z \bullet n) = 8$$

$x \bullet y \quad \in NI[4 \leq x \bullet y \leq 8]$, N est l'ensemble des nombres naturels,
$x \qquad \in ZI[4 \leq x \leq 6]$, Z est l'ensemble des nombres entiers,
$z \qquad \in NI[0 \leq z \leq 4]$,
$n \qquad \in ZI[1 \leq n \leq 3]$,

R est la fraction oligosaccharide du sucre, de préférence un mono-, di- ou trisaccharide, d'un amino glycoside et M est un élément capable de former un cation pharmaceutiquement acceptable, de préférence un élément sélectionné à partir du groupe consistant en, les métaux alcalins, les métaux alcalino terreux et l'aluminium, plus préférentiellement Na, K, Mg, Ca, Al, $M_z^{n+}$ ou désigne l'ammonium $NH_4^+$.

2. Composé selon la revendication 1, dans lequel M est un élément sélectionné à partir du groupe consistant en, les métaux alcalins, les métaux alcalino terreux et l'aluminium.

3. Composé selon la revendication 1, dans lequel l'amino glycoside est sélectionné à partir du groupe consistant en, la kanamycine A, la gentamycine, la néomycine, la nétilmycine, la streptomycine, la tobramycine.

4. Composé selon l'une quelconque des revendications 1, 2 ou 3, sélectionné à partir/parmi les composés présentant les formules suivantes :

[acide sucrose-octa-O-sulfonique] - [kanamycine A]$_2$,

[acide sucrose-octa-O-sulfonique] - [gentamycine]$_2$,

[acide sucrose-octa-O-sulfonique] - [néomycine]$_{1.3}$,

[acide sucrose-octa-O-sulfonique] - [nétilmycine]$_{1.6}$,

[acide sucrose-octa-O-sulfonique] - [streptomycine]$_{1.6}$,

[acide sucrose-octa-O-sulfonique] - [tobramycine]$_{1.6}$,

et des sels pharmaceutiquement acceptables de ceux-ci.

5. Composé selon la revendication 1 sous la forme d'un sel d'acide sucrose-octa-O-sulfonique avec un amino glycoside antimicrobien actif et avec de l'aluminium.

6. Composé selon la revendication 5 dans lequel les liaisons chimiques entre l'acide sucrose-octa-O-sulfonique, un amino glycoside et l'aluminium sont des liaisons ioniques.

7. Composition selon la formule :

$$([acide\ sucrose\text{-}octa\text{-}O\text{-}sulfonique}^{8-}] - [R\text{-}(NH_3^+)_x]_y - [Al_n(OH)_{3n\text{-}1}^+]_z,$$

dans laquelle

$$(x \bullet y) + z = 8$$

$x \bullet y \ \in NI[1{\leq}x{\bullet}y{\leq}6]$, N est l'ensemble des nombres naturels,
$x \ \ \ \ \in ZI[4{\leq}x{\leq}6]$, Z est l'ensemble des nombres entiers,
$z \ \ \ \ \in NI[2{\leq}z{\leq}7]$,
$n \ \ \ \ \in ZI[1{\leq}n{\leq}5]$,

dans laquelle R est la fraction oligosaccharide du sucre, de préférence un mono-, di- ou trisaccharide, d'un amino glycoside antimicrobien actif.

8. Composition selon la revendication 1 de formule :

$$(C_{12}H_{14}O_{11}[(SO_3^-)_8\text{-}R\text{-}(NH_3^+)_s\text{-}(Al_2(OH)_5^+)_t]),$$

dans laquelle $1 \leq s \leq 7$, $1 \leq t \leq 7$, et $s + t = 8$, R est la fraction oligosaccharide du sucre, de préférence un mono-, di- ou trisaccharide, d'un amino glycoside antimicrobien actif.

9. Composé selon les revendications 5, 6, 7 ou 8 dans lequel l'amino glycoside est sélectionné à partir du groupe consistant en la kanamycine A, la gentamycine, la néomycine, la nétilmycine, la streptomycine, la tobramycine.

10. Composé selon les revendications 5, 6, 7 ou 8 dans lequel l'amino glycoside est la tobramycine.

11. Procédé pour produire les composés selon les revendications 1 à 4, dans lequel une solution aqueuse de base d'amino, glycoside est mise à réagir avec une solution aqueuse d'acide sucrose-octa-O-sulfonique.

12. Procédé selon la revendication 11, dans lequel les cations, de préférence $K^+$, $Na^+$, $Mg^{2+}$, $Ca^{2+}$ et $Al^{3+}$, sont introduits pour obtenir des sels qui sont pharmaceutiquement acceptables.

13. Procédé de production des composés selon les revendications 1 à 4, dans lequel une solution aqueuse d'un sel d'amino glycoside résultant de l'addition d'un acide est mise à réagir avec une solution aqueuse d'un sel d'acide sucrose-octa-O-sulfonique suivie par une dialyse afin de supprimer les sels inorganiques.

**14.** Procédé de production d'un composé selon les revendications 5 à 10, dans lequel une suspension aqueuse de sucralfate (« sucralfate ») est mise à réagir avec un amino glycoside dans l'eau à un pH d'environ 4.

**15.** Composition pharmaceutique contenant au moins un composant présentant la formule générale I comprenant de 50 à 250 mg d'un amino glycoside antimicrobien actif par dose unitaire, et une base (« carrier ») acceptable parmaceutiquement.

**16.** Composition pharmaceutique contenant au moins un des composants selon la revendication 5 comprenant de 50 à 250 mg d'un amino glycoside antimicrobien actif par dose unitaire, et une base acceptable parmaceutiquement.

**17.** Composé pour le traitement des gastrites et des ulcérations de l'estomac et/ou du duodénum chez un sujet ayant besoin d'un tel traitement, ledit composant présentant la formule générale I :

$$([\text{acide sucrose-octa-O-sulfonique}^{8-}] - [R\text{-}(NH_3^+)_x]_y\text{-}M_z^{n+}) \qquad (I)$$

dans laquelle

$$(x \bullet y) + (z \bullet n) = 8$$

$x \bullet y \quad \in NI[4{\leq}x{\bullet}y{\leq}8]$, N est l'ensemble des nombres naturels,
$x \qquad \in ZI[4{\leq}x{\leq}6]$, Z est l'ensemble des nombres entiers,
$z \qquad \in NI[0{\leq}z{\leq}4]$,
$n \qquad \in ZI[1{\leq}n{\leq}3]$,

dans laquelle R est la fraction oligosaccharide du sucre, de préférence un mono-, di- ou trisaccharide, d'un amino glycoside et M est un élément capable de former un cation pharmaceutiquement acceptable, de préférence un élément sélectionné à partir du groupe consistant en, les métaux alcalins, les métaux alcalino terreux et l'aluminium, plus préférentiellement Na, K, Mg, Ca, Al, ou $M_z^{n+}$ désignent l'ammonium $NH_4^+$ et des sels pharmaceutiquement acceptables de ceux-ci, dont le composé est efficace pour une telle application.

**18.** Composé selon la revendication 5 et des sels pharmaceutiquement acceptables de celui-ci pour le traitement de gastrites et d'ulcérations de l'estomac et/ou du duodénum chez un sujet nécessitant un tel traitement, lequel composé est efficace pour un tel traitement.

**19.** Composé pour le traitement de gastrites et d'ulcérations dans l'estomac et/ou le duodénum chez un sujet nécessitant un tel traitement, ledit composant présentant la formule générale :

$$([\text{acide sucrose-octa-O-sulfonique}^{8-}]\text{-}[R\text{-}(NH_3^+)_x]_y - [Al_n(OH)_{3n-1}^+]_z,$$

dans laquelle

$$(x \bullet y) + z = 8$$

$x \bullet y \quad \in NI[1{\leq}x{\bullet}y{\leq}6]$, N est l'ensemble des nombres naturels,
$x \qquad \in ZI[4{\leq}x{\leq}6]$, Z est l'ensemble des nombres entiers,
$z \qquad \in NI[2{\leq}z{\leq}7]$,
$n \qquad \in ZI[1{\leq}n{\leq}5]$,

dans laquelle R est la fraction oligosaccharide du sucre, de préférence un mono-, di- ou trisaccharide, d'un amino glycoside antimicrobien actif, et des sels pharmaceutiquement acceptables de ceux-ci, lequel, composant est efficace pour un tel traitement.

**20.** Composé pour le traitement de gastrites et d'ulcérations dans l'estomac et/ou le duodénum chez un sujet nécessitant un tel traitement, ledit composant présentant la formule :

$$(C_{12}H_{14}O_{11}[(SO_3^-)_8\text{-}R\text{-}(NH_3^+)_s\text{-}(Al_2(OH)_5^+)_t]),$$

dans laquelle $1 \leq s \leq 7$, $1 \leq t \leq 7$, et $s + t = 8$, R est la fraction oligosaccharide du sucre, de préférence un mono-, di- ou trisaccharide, d'un amino glycoside antimicrobien actif.

21. Composé selon les revendications 19, 20, 21 ou 22, dans lequel ledit composant se trouve sous la forme d'une composition pharmaceutique comprenant de 50 à 250 mg d'un amino glycoside antimicrobien actif par dose unitaire en présence d'une base pharmaceutiquement acceptable ou d'un diluant.

22. Utilisation d'un composé selon l'une quelconque des revendications 1 à 10 pour la préparation d'un médicament convenable pour le traitement de gastrites et d'ulcérations dans l'estomac et le duodénum chez les mammifères.

23. Utilisation d'un composé selon l'une quelconque des revendications 1 à 10 pour la préparation d'un médicament convenable pour l'éradication d'Helicobacter pylori dans l'estomac et le duodénum d'un mammifère.

**Fig. 1**
Tobramycin/1.94mg
Heat Flow (mW)

# Fig. 2
## Tobramycin-sos/3.33mg
### Heat Flow (mW)

EP 0 725 786 B1

# Fig. 3

Tobramycin Sigma T-4014

EP 0 725 786 B1

# Fig. 4

## TOBRAMYCIN-OCTA-O-SULFONSYRE

EP 0 725 786 B1

Fig. 5

STREPTOMYCIN-SUCROSE-OCTA-O-SULFONSYRE

23

Fig. 6